# DEMANDE DE BREVET EUROPEEN

(11) **EP 0 624 653 A1**
(43) Date de publication de la demande: **17.11.1994**
(21) Numéro de dépôt: 94106826.4
(22) Date de dépôt: 02.05.1994
(51) Int. Cl.: C12Q 1/00, G01N 33/487

(54) **Capteur électro-chimique à zones multiples sur disque et son application au dosage du glucose**

(30) Priorité: 10.05.1993 FR 9305683
(71) Demandeur: ASULAB S.A., CH-2501 Bienne (CH)
(72) Inventeur: Saurer, Eric, CH-2022 Bevaix (CH)
(74) Mandataire: Thérond, Gérard Raymond

(57) **Abrégé**

Capteur électrochimique comportant des zones de mesure multiples (34) séquentiellement isolables, disposées sur le pourtour d'un disque isolant (1), reliées entre elles par des collecteurs de courant (16, 17), jusqu'à des zones centrales formant les contacts (23, 24) avec un appareil de mesure.

Le disque-contact comporte également un dispositif d'entraînement du disque en rotation (6), des repérages de position (11) et d'isolement (7) des zones de mesure (34) et peut être contenu dans un boîtier (40). Application au dosage du glucose.

## Description

La présente invention a pour objet un capteur électrochimique comportant, sur un même support mince isolant rigide en forme de disque de petite dimension, plusieurs zones actives traversées par un même jeu d'électrodes, permettant d'effectuer plusieurs mesures successives d'une même donnée au moyen d'un unique capteur introduit dans un appareil de mesure approprié.

La présente invention a plus particulièrement pour objet un capteur ampérométrique destiné à mesurer le taux de glucose dans le sang.

De nombreuses techniques électrochimiques ont été développées pour détecter au moyen d'électrodes et d'un circuit électrique ou électronique approprié les caractéristiques physico-chimiques d'un milieu, ou pour déterminer les concentrations de substances présentes dans des solutions, des effluents, ou des fluides d'origine naturelle, biochimique ou biologique. Ces techniques ont en commun l'utilisation d'au moins deux électrodes pour effectuer des mesures par conductimétrie, voltamétrie, ampérométrie ou polarographie, en faisant intervenir, si nécessaire, un réactif ajouté au milieu, ou confiné au voisinage d'une électrode.

Pour le dosage du glucose dans le sang, le réactif précité comprendra au moins une enzyme spécifique du glucose, associé de préférence avec un médiateur.

A la génération des appareils de laboratoire encombrants, couteux et utilisables seulement par un personnel qualifié, ont succédé des appareils de plus petite dimension, voire portable, munis de dispositifs d'électrodes désignés sous le terme général de "capteur"

Les premiers capteurs électrochimiques, de petite dimension et séparables de l'appareil de mesure, donc jetables, ont, semble-t-il été décrits par Miles Laboratories Inc. dans la demande de brevet DE 2 127 142 revendiquant une priorité de juin 1970. Le dispositif décrit dans cette demande concerne un capteur électrochimique dans lequel deux électrodes sont disposées à plat sur une plaquette isolante de petite dimension par une technique de déposition dite en couche mince.

On peut également citer les nombreuses publications de C.C.LIU, M.R. NEUMAN et al (Marine Technology 16 (1980) 468-472; Diabete Care Vol. 5, No 3, May-June 1982) concernant des recherches effectuées en vue de perfectionner les capteurs miniatures et permettre notamment le dosage du glucose dans le sang avec des capteurs pouvant être jetés après chaque usage.

De nombreux autres documents décrivent des modes particuliers de réalisation de tels capteurs, ne comportant toujours qu'une seule zone de mesure, connectables à un appareil de mesure et jetable après le premier usage. Le brevet EP 0 406 304 décrit par exemple un capteur muni d'électrodes superposées.

Lorsqu'on a plusieurs zones de mesure, il s'agit toujours d'une juxtaposition de plusieurs capteurs discrets, chaque capteur étant pourvu de son propre système de connexion à un appareil de mesure. Un exemple de ce type de réalisation est donné dans le brevet EP 0 170 375.

La demande de brevet FR 93 01331 au nom de la demanderesse, incorporée par référence à la présente demande, constitue une étape supplémentaire dans l'évolution et dans le principe des capteurs précités, en ce que le capteur décrit est constitué par une succession de zones actives disposées sur un substrat isolant en forme de bande, lesdites zones actives étant toutes reliées entre elles jusqu'à une zone de connexion commune avec l `appareil de mesure, chaque zone étant séquentiellement détachable de la bande support après que la mesure ait été effectuée. Par rapport aux capteurs antérieurement connus, un tel dispositif offre de nombreux avantages pour l'utilisateur qui peut effectuer plusieurs mesures avant de mettre en place un nouveau capteur dans l'appareil. Il est toutefois évident que le nombre de mesures que l'utilisateur peut effectuer avec un seul capteur, lorsque celui-ci comporte un substrat en matériau rigide, est limité par les dimensions de l'appareil de mesure destiné à recevoir ledit capteur. Si l'on souhaite que l'appareil de mesure puisse être transporté dans une poche de vêtement, la plus grande dimension de l'appareil de mesure, et donc du capteur en bande, sera de l'ordre de 15 cm et ledit capteur comportera alors au plus cinq à six zones actives alignées sur une même bande.

Un des buts de l'invention est au contraire de procurer un capteur comportant également plusieurs zones actives, lesdites zones n'étant plus alignées, mais disposées sur une couronne externe d'un disque rigide de petite dimension en matériau isolant, tout en ayant une connexion commune à la proximité du centre du disque, ladite connexion permettant le raccordement à un appareil de mesure pendant la durée d'utilisation de toutes les zones actives. Un tel capteur selon l'invention sera par la suite désigné par le terme général "disque-capteur".

Un autre but de l'invention est de procurer un capteur possédant, pour un diamètre donné, un plus grand nombre de zones actives qu'un capteur multizones en bande ayant une longueur identique au diamètre du capteur selon l'invention.

Un autre but de l'invention est de fournir un capteur dont les zones actives sont séquentiellement isolables après chaque mesure des autres zones actives non encore utilisées, sans qu'il soit nécessaire de les détacher entièrement du disque support.

Un autre but de l'invention est de fournir un dispositif capteur dans lequel le disque-capteur est protégé par un boîtier enveloppant le disque et facilitant en outre sa manipulation, sa mise en place dans l'appareil de mesure, ainsi que sa connexion électrique au circuit électronique de l'appareil de mesure, son entraînement en rotation pour positionner et présenter les zones de mesure successives, et l'isolation des zones de mesure utilisées des autres zones de mesure non encore utilisées.

Le disque-capteur à zones actives multiples séquentiellement isolables selon l'invention est constitué par un disque rigide en un matériau isolant de faible épaisseur sur lequel au moins deux collecteurs de courant en matériaux conducteurs, électriquement isolés, sont disposés sur une même face ou sur les faces opposées pour constituer une zone de connexion près du centre du disque et pour relier les zones actives situées à sa périphérie en constituant dans ces zones les électrodes qui permettent d'effectuer les mesures électrochimiques.

Le matériau utilisé pour former le disque isolant rigide sera par exemple une résine synthétique telle qu'une résine vinylique, une céramique, le mica, un polyéthylène terephtalate ou le verre; selon la matériau isolant utilisé, on donne au disque la forme désirée par moulage, estampage, découpage ou tout autre procédé approprié. Pour pouvoir être utilisé dans un appareil de mesure facilement portable dans la poche d'un vêtement, le disque a une épaisseur comprise entre 0,5 et 6 mm et un diamètre compris entre 5 et 15 cm, de préférence entre 6 et 12 cm. Il est bien évident que le diamètre du disque sera également fonction du nombre de zones actives souhaitées sur une même disque, sachant qu'une distance minimale d'environ 10 à 20 mm est nécessaire pour éviter qu'une zone de mesure venant d'être utilisée ne perturbe la ou les zones de mesure non encore utilisées. Il est ainsi possible de réaliser des disques-capteurs comportant entre 6 et 24 zones actives, et de préférence entre 8 et 12 zones actives. Pour un disque ayant un diamètre d'environ 7 cm, il sera par exemple possible de disposer 8 zones de mesures sur une même face du disque.

Les collecteurs de courant sont disposés sur une face ou sur les deux faces du disque selon une configuration définie, par des techniques connues de déposition, dites en couche mince ou épaisse, du matériau conducteur approprié; ces collecteurs peuvent également être disposés sur le disque par laminage d'un film isolant revêtu dudit matériau conducteur. A titre de matériau conducteur approprié, on utilisera par exemple l'or, l'argent, le platine, le nickel, la palladium, le titane ou le carbone.

Lorsque le disque-capteur selon l'invention est destiné au dosage du glucose dans le sang, un collecteur de courant comporte des portions constituant l'électrode de référence. Le matériau conducteur utilisé pour l'électrode de travail est l'un des métaux précités, ainsi que le carbone; le matériau conducteur de l'électrode de référence est généralement de l'argent recouvert d'une couche de chlorure d'argent. Selon des méthodes bien connues de l'art antérieur, telles que celles décrite dans la demande WO 92/14 836, l'électrode de travail comporte en outre un réactif comprenant au moins une enzyme d'oxydoréduction spécifique du glucose (GOD) et au moins un médiateur permettant le transfert des électrons entre ladite enzyme et ledit collecteur de courant. A titre de médiateur approprié, on utilise par exemple un complexe d'un métal de transition avec au moins un ligand bipyridine, terpyridine ou phénanthroline substitué par au moins un groupe donneur d'électrons, tels que ceux décrits dans le brevet WO 92/14 741.

Lorsque les collecteurs de courant sont supportés par une même face du disque, ils se présentent alors sous forme de deux anneaux concentriques, isolés électriquement l'un de l'autre, situés à l'intérieur d'une couronne étroite à la périphérie du disque, un anneau au moins situé le plus près du centre étant ouvert, les deux anneaux étant reliés par des extensions radiales des collecteurs de courant à la zone centrale où s'effectue la connexion avec un appareil de mesure. Les zones actives sont réparties dans la couronne périphérique, dans des secteurs égaux et sont délimitées au droit des conducteurs de courant, soit par déformation du disque support isolant, soit par des fenêtres ménagées dans un film de recouvrement isolant. Les portions de collecteurs de courant situées à l'intérieur des zones ainsi délimitées constituent les électrodes de chaque zone active.

Lorsque le disque support isolant et les collecteurs de courant sont revêtus d'un film de recouvrement isolant, ce dernier comporte également des fenêtres rendant accessibles les zones de contact situées près du centre et des fenêtres entre chaque zone active laissant apparaître des portions des deux collecteurs qui seront griffés ou découpés, par un dispositif intégré à l'appareil de mesure, de façon à isoler une zone active utilisée des autres zones actives non encore utilisées.

Les zones de connexion situées près du centre du disque, et prolongeant les extrémités des extensions des collecteurs de courant, sont constituées soit par des petites surfaces en forme de pastilles, soit par une couronne ou un disque concentrique.

Le disque capteur est également pourvu de moyens d'entraînement en son centre, à sa périphérie ou sur l'une de ses faces.

Lorsque l'organe d'entraînement est situé au centre, cela permet avantageusement à l'organe d'entraînement associé de l'appareil de mesure d'assurer également la connexion avec les zones de contact des conducteurs de courant, que ces zones soient en forme de couronne, ou en forme de pastille aux extrémités des extensions des collecteurs de courant. Cet organe d'entraînement central sera avantageusement constitué par un trou traversant le disque ayant pour section un polygone régulier, ou un polygone irrégulier dans le cas où le mode réalisation du disque-capteur nécessite un repérage de la première zone active à utiliser.

Lorsque l'organe d'entraînement est situé à la périphérie, il est par exemple constitué par une dentelure du bord du disque capteur, destiné à venir coopérer avec une roue dentée de l'appareil de mesure.

Lorsque l'organe d'entraînement est situé sur une face du disque, il pourra se situer sur la face comportant les zones actives si le disque-capteur comporte un revêtement isolant couvrant les conducteurs de courant, mais il sera plus avantageusement situé sur la face opposée. Cet organe d'entraînement sera par exemple constitué, par une couronne crantée ou par une roue de friction formée en même temps que le disque ou rapportée.

Dans le cas où l'organe d'entraînement ne permet pas en même temps d'assurer le centrage du disque-capteur dans l'appareil de mesure, des moyens supplémentaires sont prévus, telle qu'une bague ou un axe. Le centrage peut également être effectué, si le mode de réalisation du disque-capteur le permet, au moyen même du logement destiné à recevoir ledit disque.

Pour éviter qu'une même zone ne soit utilisée deux fois de suite, le mécanisme d'entraînement de l'appareil de mesure peut être conçu à cet effet.

Le disque-capteur lui-même peut également très simplement être pourvu, à sa périphérie ou sur l'une de ses faces, d'un cliquet anti-retour et d'une portion de nervure radiale empêchant le disque d'effectuer plus d'une révolution. La portion de nervure radiale peut également constituer l'organe d'entraînement du disque-capteur, en coopérant avec une rainure d'une pièce rotative de l'appareil de mesure.

De même, le dispositif d'entraînement de l'appareil de mesure peut être conçu pour que chaque manipulation n'entraîne le disque-capteur que d'un pas correspondant au passage d'une zone active à l'autre. Le disque-capteur peut également lui-même posséder des organes de positionnement constitués par des encoches pratiquées dans le bord du disque, entre chaque zone active et avec le même pas que celui existant entre une zone active et la suivante.

Le nombre de zones actives portées par une face du disque sera évidemment fonction du diamètre du disque lui-même. Comme on le verra dans les exemples qui vont suivre, pour un disque capteur de petite dimension, c'est-à-dire pour un appareil de mesure portable dans une poche de vêtement, ce nombre pourra varier de 5 à 18, voire davantage si le mode de réalisation permet d'utiliser les deux faces du disque.

Lorsque les collecteurs de courant sont disposés sur les faces opposées du disque, ils sont répartis en étoile à partir d'un disque ou d'une couronne centrale constituant le zone de contact. Les zones de mesure se trouvent alors tout à fait à la périphérie. Les modes de réalisation qui ont précédemment été mentionnés pour un disque-capteur ayant les deux collecteurs de courant sur une même face sont également applicables à un disque-capteur ayant les collecteurs sur chaque face, en excluant toutefois un dispositif d'entraînement par dentelure du bord. Lorsque les conducteurs de courant sont disposés en étoile sur chaque face du disque, les zones actives peuvent en outre être isolées après chaque mesure en les brisant selon un contour préformé. Dans un tel mode de réalisation le disque-capteur comportera par exemple à sa périphérie des échancrures radiales s'étendant jusqu'à une zone circulaire affaiblie par une rainure.

Pour protéger les zones actives et pour faciliter la manipulation du disque-capteur et sa mise en place dans l'appareil de mesure, le disque-capteur peut être contenu dans un boîtier de forme ronde, carrée ou ayant une forme combinant ces deux formes. Le boîtier est pourvu d'une ouverture donnant accès à une seule zone active, d'une ouverture permettant le griffage des collecteurs de courant, d'une ou de deux ouvertures donnant accès aux zones de contact et, si nécessaire, d'une ouverture supplémentaire donnant accès au dispositif d'entraînement. Le boîtier comporte également sur ses faces internes des organes venant coopérer avec les organes correspondants du disque-capteur pour contribuer à son centrage. Le boîtier comporte également sur une face interne, ou sur les deux selon le mode de réalisation, une couronne continue ou discontinue destinée à venir en appui sur une seule face du disque-capteur, ou sur les deux selon le mode de réalisation, de façon à guider le disque-capteur en rotation, tout en le maintenant légèrement écarté des parois internes inférieures et supérieures du boîtier. Lorsque le boîtier est parfaitement rond une nervure peut être prévue sur l'une de ses faces externes pour faciliter le positionnement dans l'appareil de mesure.

L'invention sera mieux comprise en référence aux exemples de réalisation tels que ceux représentés par les dessins annexés dans lesquels
- la figure 1 représente une vue de dessus d'un premier mode de réalisation d'un disque-capteur selon l'invention;
- la figure 2 représente une coupe diamétrale de la figure 1 selon la ligne II-II;
- la figure 3 représente une coupe diamétrale partielle agrandie à travers le centre d'une zone active selon la ligne III-III;
- la figure 4 représente une vue de dessus d'un deuxième mode de réalisation, dans lequel le revêtement supérieur est partiellement arraché;
- la figure 5 représente une coupe diamétrale de la figure 4 selon la ligne V-V;
- la figure 6 représente une autre coupe diamétrale de la figure 4 selon la ligne VI-VI;
- la figure 7 représente une vue de dessus d'un troisième mode de réalisation dans lequel le revêtement supérieur du disque-capteur est partiellement arraché.
- la figure 8 représente une coupe diamétrale de la figure 7 selon la ligne VIII-VIII;
- la figure 9 représente une vue de dessous de la figure 7 dans laquelle une partie du revêtement isolant a été arrachée;
- la figure 10 représente un quatrième mode de réalisation dans lequel une partie du boîtier et du revêtement du disque-capteur sont partiellement arrachés;
- la figure 11 représente une coupe diamétrale de la figure 10 selon la ligne XI-XI;
- la figure 12 représente un cinquième mode de réalisation dans lequel une partie du boîtier et du revêtement du disque-capteur sont partiellement arrachés;
- la figure 13 représente une coupe diamétrale de la figure 12 selon la ligne XIII-XIII;
- la figure 14 représente un sixième mode de réalisation dans lequel une partie du boîtier et du revêtement du disque-capteur sont partiellement arrachés.
- la figure 15 représente une coupe diamétrale de la figure 14 selon la ligne XV-XV;
Dans toutes les représentations en coupe, pour faciliter la compréhension de l'agencement des différentes couches, les proportions entre les épaisseurs des différentes couches et leur surface ne sont pas respectées; de même, les parties arrières des revêtements isolants arrachés ne sont pas représentées.

Dans toutes les figures les parties similaires du disque-capteur ou du boîtier sont désignées par la même référence, quelque soit le mode de réalisation.

Dans les figures comportant des éléments répétitifs, seuls un ou deux éléments comportent une référence.

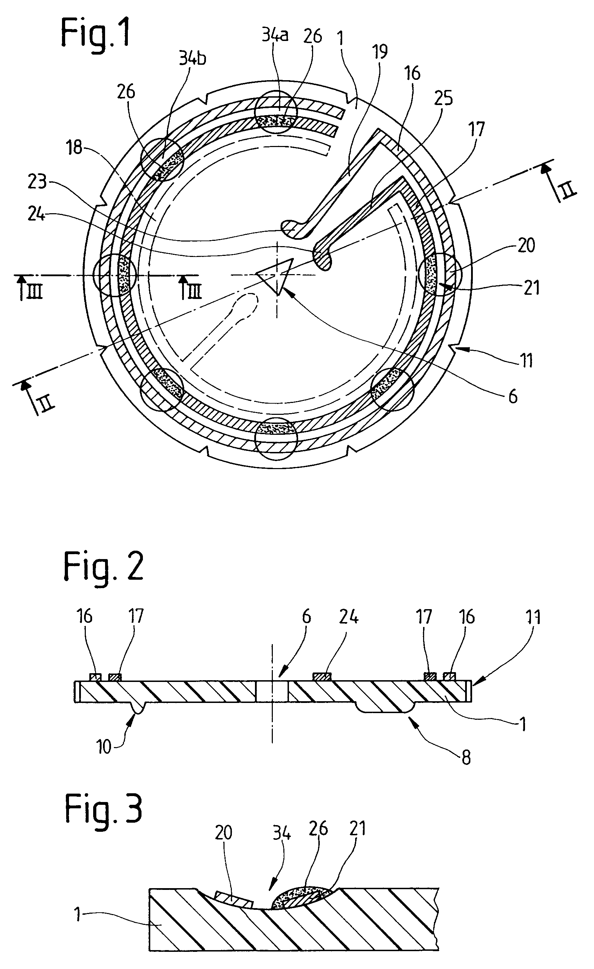

Selon un premier mode de réalisation, représenté aux figures 1 à 3, le capteur est constitué par un disque support 1 en matériau isolant d'un épaisseur de l'ordre de 1 mm et d'un diamètre de 7 cm. Ce disque est préformé par emboutissage pour comporter à sa périphérie des encoches de positionnement 11 régulièrement espacées, d'un pas de 45° définissant ainsi 8 secteurs circulaires égaux. A l'intérieur de 7 de ces secteurs, dans leur axe médian et près de la périphérie sont délimitées les zones qui constituent les zones de mesure 34. En son centre, le disque 1 est percé d'un trou 6 en forme de triangle isocèle ayant son axe de symétrie confondu avec celui du huitième secteur circulaire. Sur la surface de ce disque, et près de sa périphérie sont disposés deux collecteurs de courant 16, 17 représentés pour simplifier sous forme de deux anneaux étroits concentriques ouverts, dont chacun possède à l'une de ses extrémités une extension radiale 19 ou 25, dirigée vers le centre, dans le secteur circulaire n'ayant pas de zone de mesure 34, où elle se termine par une petite surface en forme de pastille constituant une zone de contact 23 ou 24. En partant à proximité du centre, où ils seront reliés à l'appareil de mesure par les zones de contact 23, 24, les collecteurs de courant 16, 17 traversent donc successivement les 7 zones de mesure 34 à l'intérieur desquelles ils constituent les électrodes 20, 21.

Lorsque le disque-capteur est destiné à effectuer des mesures du taux de glucose dans le sang chaque zone de mesure 34 comporte en outre un réactif 26 déposé sur l'électrode de travail 21, par des techniques connues de pipetage, sérigraphie ou décalque, comme cela est représenté dans la vue agrandie de la figure 3. On conçoit aisément aussi que les zones de mesure 34 puissent comporter entre les électrodes 20 et 21 une petite surépaisseur obtenue au formage du disque pour faciliter la déposition du réactif 26. En se référant à la figure 1, on observe aussi que le disque 1 peut supporter plus de deux électrodes, par exemple une troisième électrode de référence 18 représentée en pointillés, conformée comme les deux électrodes 16, 17 en un anneau ouvert, ayant en un point quelconque dudit anneau une extension radiale dirigée vers le centre et comportant à son extrémité une petite surface en forme de pastille formant une zone de contact.

La figure 2 est une coupe montrant deux éléments caractéristiques de la face du disque 1 ne supportant pas les collecteurs de courant. La référence 10 représente en coupe un des bossages situé sur la circonférence d'un cercle de façon à faciliter la libre rotation du disque dans l'appareil de mesure; cette succession de bossage peut également être remplacée par une nervure circulaire continue. La référence 8 représente une portion de nervure radiale destinée d'une part à positionner la première zone de mesure à utiliser 34a, d'autre part à limiter la rotation du disque 1 à une révolution, en coopération avec une nervure correspondante située dans l'appareil de mesure, de façon à interdire qu'une zone de mesure ne soit à nouveau présentée pour une nouvelle mesure. L'entraînement du disque peut s'effectuer par un organe de l'appareil de mesure venant coopérer soit avec le trou 6, soit avec la nervure 8.

La séparation d'une zone de mesure usagée - par exemple 34a - d'une zone de mesure non utilisée - par exemple 34b - est effectuée par un organe de l'appareil de mesure lui-même venant griffer les deux collecteurs 16, 17 de courant ou sectionner le disque le long de l'un de ses rayons entre les deux zones 34a et 34b, à un endroit repéré grâce aux encoches 11.

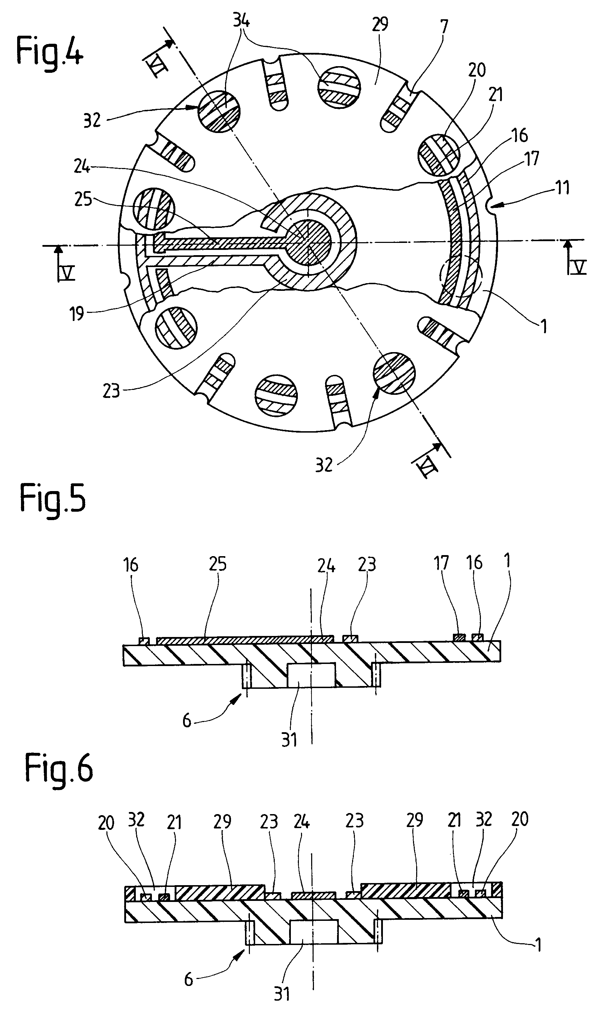

Le disque-capteur correspondant à une deuxième variante représentée aux figures 4, 5 et 6, comporte de la même façon un disque 1 divisé en 8 secteurs circulaires égaux, chacun des 8 secteurs étant occupés par une zone de mesure 34, chaque zone de mesure étant délimitée par une fenêtre de même dimension ménagée dans un film de recouvrement isolant 29. Outre les fenêtres correspondant aux zones de mesures, ledit film de recouvrement comporte également une fenêtre 32 au niveau des zones de contact 23, 24 et des fenêtres 7 régulièrement espacées près de la périphérie du disque, et découvrant de petites portions des collecteurs 16, 17 qui pourront être griffés dans ces zones pour isoler une zone de mesure usagée. Grâce au film de recouvrement isolant 29 les extensions radiales 19, 25 peuvent être rapprochées d'une zone de mesure, et être situées dans un secteur circulaire plus petit compris entre une zone de mesure 34 et une fenêtre 7, en permettant ainsi l'occupation de tous les secteurs circulaires par 8 zones de mesure. Selon le mode de réalisation de cette variante, les zones de contact 23, 24 se composent d'un disque situé au centre et d'un anneau concentrique ouvert. Le dispositif d'entraînement 6 sera alors constitué, par exemple, par un pignon situé sur la face du disque ne supportant pas les collecteurs et rapporté par collage ou issu du formage du disque.

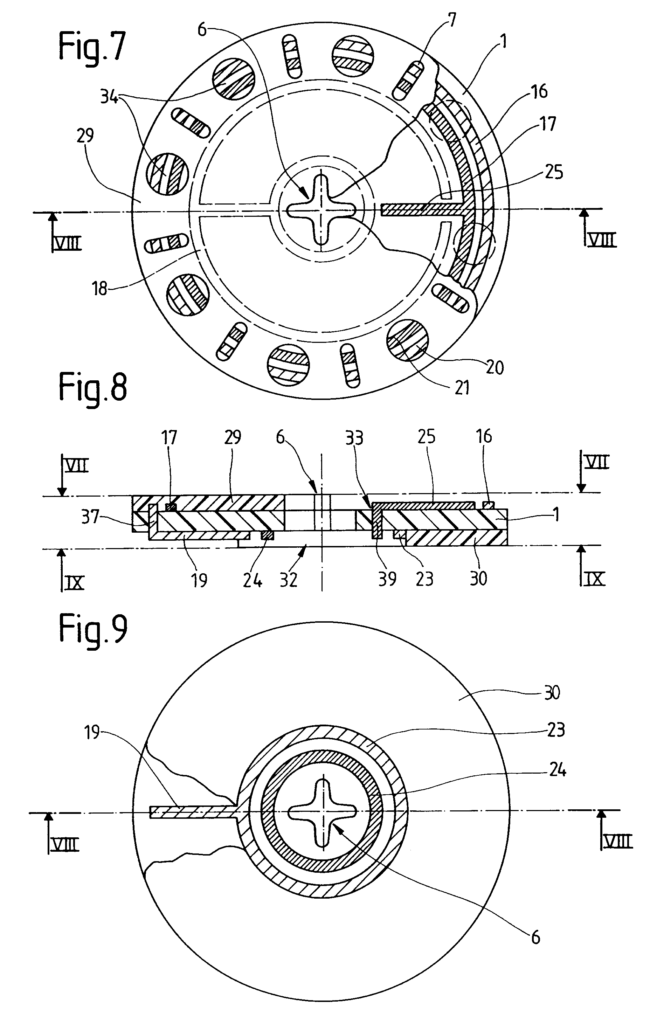

Les figures 7, 8 et 9 représentent une troisième variante comportant sur une face supérieure toutes les zones de mesure 34 et sur l'autre face les zones de contact 23, 24, chaque face comportant un film de recouvrement isolant 29, 30. A cet effet, l'extension radiale 25 du collecteur de courant 17, en forme d'anneau fermé situé le plus près du centre, possède une petite portion traversante 39 rejoignant par un passage 33 une zone de contact 24, située sur l'autre face du disque, en forme d'anneau fermé situé près du centre du disque. De même, le collecteur de courant 16, en forme d'anneau fermé situé le plus près de la périphérie sur la face supérieure du disque, possède une petite portion traversante 37, rejoignant l'extension radiale 19 située sur l'autre face, ladite extensions étant terminée par une zone 23 ayant la forme d'un anneau fermé concentrique à l'anneau 24. Dans ce mode réalisation le dispositif d'entraînement 6 est constitué par un trou traversant central en forme d'étoile.

La face du disque comportant les collecteurs de courant 16, 17 peut également supporter un troisième collecteur 18, représenté en pointillés.

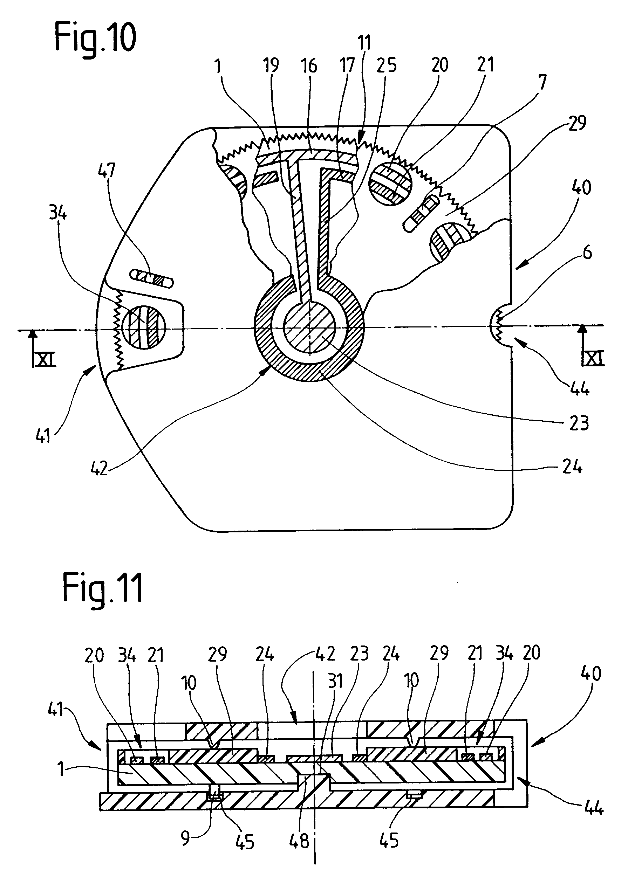

Les figures 10 et 11 représentent une quatrième variante d'un disque-capteur du type de celui représenté aux figures 4 à 6 mais comportant 12 secteurs circulaires égaux, ledit disque-capteur étant tout entier contenu dans un boîtier 40 permettant à la fois de protéger les 11 zones de mesure 34 et de faciliter sa mise en place dans l'appareil de mesure de façon à positionner correctement la première zone de mesure à utiliser, ainsi que le mécanisme d'entraînement 6, les zones de contacts 23, 24, et les zones d'isolement 7 des zones de mesure usagées. Dans la variante représentée le boîtier se compose d'un demi-disque et d'un demi-carré accolés selon un diamètre ménageant un espace à l'intérieur du boîtier à peine supérieur au diamètre du disque 1. A l'extrémité du demi-disque dans un axe perpendiculaire au plan de jonction du demi-disque et du demi-carré, le boîtier comporte une ouverture 41 donnant accès à une seule zone de mesure. A proximité de l'ouverture 41, se trouve une autre ouverture 47, positionnée pour se trouver au-dessus d'une zone d'isolement 7 la plus proche de la zone de mesure en place dans l'ouverture 47. Au centre du disque, le boîtier comporte une ouverture 42 donnant accès aux zones de contact 23, 24. Dans la variante représentée le mécanisme d'entraînement 6 du disque-capteur est constitué par une dentelure du bord du disque sur toute sa périphérie; le boîtier 40 comporte alors une autre ouverture 44 située sur le même axe que l'ouverture 41 et sur le demi-carré du boîtier. L'ouverture 41 peut également se situer à un autre endroit du pourtour du boîtier, en fonction de l'agencement du mécanisme d'entraînement de l'appareil de mesure destiné à venir coopérer avec la dentelure du disque-capteur.

La coupe de la figure 11 montre également que le boîtier 40 permet de maintenir le disque capteur dans une position spatiale prédéterminée. A cet effet, le fond du boîtier comporte en son centre un petit axe 48 venant coopérer avec un trou borgne 31 du disque pour permettre le centrage de ce dernier dans le boîtier 40. Dans le mode de réalisation représenté la libre rotation du disque dans le boîtier est assurée par la coopération des bossages 10, supportés non plus par le disque, mais par le boîtier, et du petit axe de centrage 48. Sur la coupe de la figure 11 est également représenté un dispositif anti-retour. Ce dispositif anti-retour se compose par exemple d'un petit cliquet 9 porté par le disque 1, destiné à venir coopérer avec une rainure crantée dans le fond du boîtier.

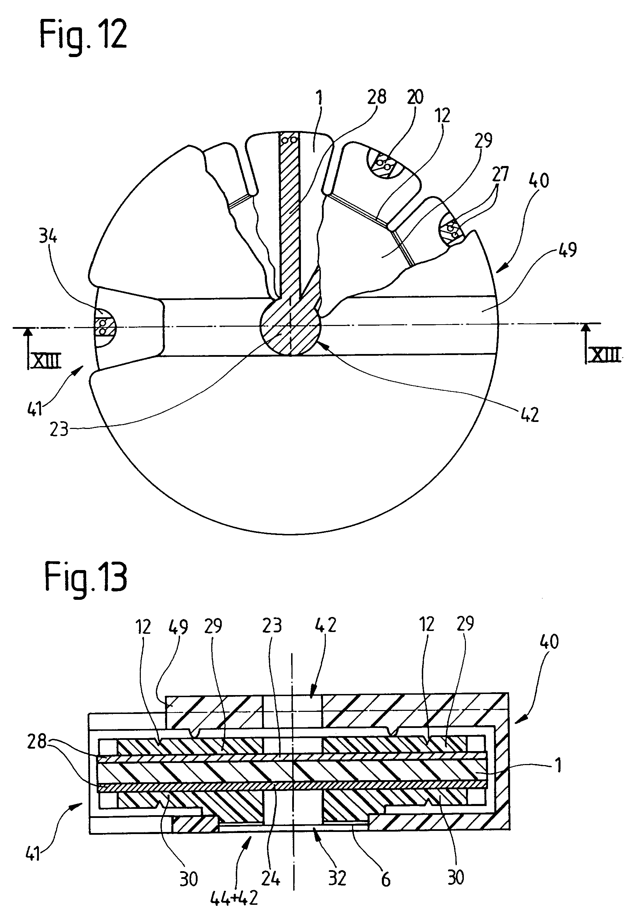

Les figures 12 et 13 représentent un cinquième mode de réalisation dans lequel un boîtier circulaire contient un disque-capteur en matériau isolant cassant dont chaque face supporte un collecteur de courant 16 ou 17, les deux collecteurs ayant la même configuration sur chaque face.

En référence a la figure 12, il apparaît que chaque collecteur à la forme d'une étoile dont chaque branche 28 part d'un disque central formant zone de contact 23 ou 24 et s'étend jusqu'au bord du disque 1 ou elle constitue une électrode 20 ou 21 d'une zone de mesure 34, le contact ionique entre les électrodes étant assuré par l'échantillon d'analyse, par mouillage autour du bord du disque et/ou par capillarité à travers de petits orifices traversants 27. Dans le mode de réalisation représenté, chaque secteur circulaire contenant une zone de mesure 34 est délimité par une échancrure radiale s'étendant du bord du disque jusqu'à une zone d'affaiblissement du revêtement 29 ou 30, permettant d'éliminer totalement une zone de mesure usagée par cassure. Le dispositif permettant à la fois le positionnement spatial du disque à l'intérieur du boîtier et son entraînement est constitué par une roue située sur une face du disque, présentant un épaulement annulaire pour le positionnement et une surface pourvue de rainures radiales destinées à venir coopérer par friction avec une couronne de friction correspondante de l'appareil de mesure.

Pour faciliter le positionnement du capteur dans l'appareil de mesure, le boîtier circulaire 40 comporte en outre, sur sa partie externe opposée à celle comportant en son centre une ouverture 44 + 42 donnant accès à la fois à une zone de contact 24 et à un dispositif d'entraînement 6, une nervure 49 située dans l'axe de l'ouverture 41 du boîtier et limitée par ladite ouverture. Cette nervure présente au centre du disque une deuxième ouverture 42 donnant accès à l'autre zone de contact 23.

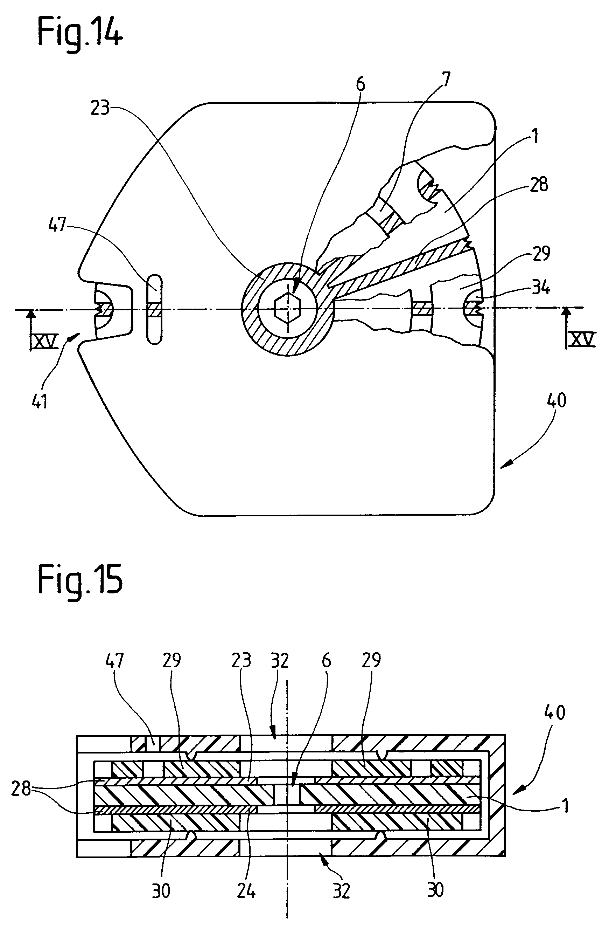

Les figures 14 et 15 représentent une sixième mode de réalisation d'un capteur, dont les collecteurs de courant sont disposés sur chaque face du disque selon le mode précédent, ledit capteur étant contenu dans un boîtier du type de celui décrit dans le quatrième mode de réalisation. Les zones de contact 23, 24 sont en forme de deux anneaux, chacun étant disposé sur une face du disque, ayant en leur centre un trou de section octogonale traversant le disque et constituant le dispositif d'entraînement 6. Les zones d'isolement 7 sont jointives et constituent une couronne où des portions de branches d'étoile 28 sont découvertes et peuvent être griffées à travers l'ouverture 47 pratiquée dans le boîtier 40.

Quel que soit le mode de réalisation, le disque-capteur selon l'invention permet, après mise en place dans un appareil de mesure, d'effectuer avec un unique capteur plusieurs mesures avant de pourvoir à son remplacement. L'appareil de mesure est pourvu de dispositifs permettant de connecter les collecteurs de courant 16, 17 à un circuit électronique de mesure et d'affichage, d'isoler électriquement les zones utilisées, d'entraîner le disque en rotation en ne laissant apparaître qu'une seule zone de mesure à la fois.

Pour les modes de réalisation illustrés dans les figures 1 à 11, l'analyse est effectuée en déposant une goutte de l'échantillon (par exemple une goutte de sang pour mesurer le taux de glucose) dans une zone de mesure 34; une fois la mesure effectuée, la zone utilisée demeure sur le disque, mais est isolée électriquement des autres zones non encore utilisées, en griffant au moyen d'un stylet solidaire de l'appareil de mesure des portions de collecteur de courant situées entre deux zones de mesure.

Pour le mode de réalisation correspondant aux figures 12 et 13 une goutte de l'échantillon à analyser est déposée sur le bord du disque, la conduction ionique entre les deux électrodes étant assurée par mouillage et/ou capillarité à travers les petits orifices traversant 27; une fois la mesure effectuée la zone utilisée peut être éliminée entièrement en cassant un fragment du disque le long de la ligne 12. On peut également, comme représenté aux figures 14 et 15 griffer une des branches 28 de l'étoile, comme indiqué pour le figures 1 à 11.

De façon générale les différents modes de réalisation décrits pour entraîner le disque-capteur en rotation, le connecter à un appareil de mesure, repérer et positionner les zones actives, peuvent donner lieu à de nombreuses autres variantes sans sortir du cadre de la présente invention.

## Revendications

1. Capteur électrochimique jetable de petite dimension comportant des zones actives multiples (34) séquentiellement séparables, permettant d'effectuer, au moyen d'un unique capteur introduit dans un appareil de mesure, plusieurs mesures successives d'une même caractéristique physico-chimique, ou d'une concentration d'un même composant présent dans une solution, un effluent ou un fluide d'origine naturelle, biologique, ou biochimique, caractérisé en ce qu'il comporte :
- un support mince isolant rigide en forme de disque (1)
- au moins deux collecteurs de courant (16, 17) en des matériaux conducteurs identiques ou différents, disposés sur le disque (1) de façon à être isolés, ayant des portions ou des extensions situées dans une étroite couronne à la périphérie du disque (1), lesdites portions ou extensions formant les électrodes (20, 21) des zones de mesure (34), reliées entre elle par lesdits collecteurs de courant (16, 17) jusqu'à une zone proche du centre du disque où leurs parties terminales sont conformées pour constituer les zones de contact (23, 24)
- des moyens d'entraînement (6) du disque (1) en rotation
- des zones de repérage (11) des zones et d'isolement (7) d'une zone active usagée des autres zones actives non encore utilisées.

2. Capteur selon la revendication 1, caractérisé en ce que les deux collecteurs de courant (16, 17) sont supportés par une même face du disque, et qu'ils sont conformés en deux anneaux concentriques disposés à l'intérieur de la couronne périphérique, l'anneau situé le plus près du centre du disque étant ouvert, chaque anneau possédant une extension radiale (19 ou 25) dirigée vers le centre du disque où il constitue une zone de contact (23 ou 24).

3. Capteur selon la revendication 1, caractérisé en ce que les deux collecteurs de courant (16, 17) sont supportés par une même face du disque et qu'ils sont conformés en deux anneaux concentriques fermés disposés à l'intérieur de la couronne périphérique, l'anneau situé le plus près du centre du disque ayant une extension radiale (25) supportée par la même face du disque et dirigée vers son centre et l'anneau le plus près du bord du disque ayant une extension radiale (19) supportée par l'autre face du disque en étant reliée audit anneau par une portion de collecteur de courant (37) traversant le disque.

4. Capteur selon la revendication 1, caractérisé en ce que les deux collecteurs de courant (16, 17) sont supportés respectivement par chaque face du disque et sont chacun conformés en étoile dont chaque branche (28) part d'un disque ou d'une couronne centrale formant zone de contact (23 ou 24) et s'étend jusqu'au bord du disque (1).

5. Capteur selon la revendication 1, caractérisé en ce que les zones de contact (23,24) sont conformés en pastille, disque ou anneau ouvert ou fermé situé sur une même face au disque, ou respectivement sur les faces opposées.

6. Capteur selon les revendications 2 ou 3, caractérisé en ce qu'il comporte un troisième collecteur de courant (18), ayant une portion concentrique proche des deux premiers collecteurs (16,17) et une extension radiale dirigée vers le centre où il forme un troisième contact.

7. Capteur selon la revendication 1, caractérisé en ce que les zones de mesure (34) sont régulièrement réparties sur le disque dans tout ou partie de secteurs circulaires égaux, et en ce que le périmètre desdites zones de mesure est délimitée, soit par une déformation du disque support isolant, soit par des fenêtres pratiquées dans un film isolant (29) recouvrant le disque.

8. Capteur selon la revendication 4, caractérisé en ce que les zones de mesure (34) comportent de petits orifices traversants (27) favorisant par capillarité la jonction ionique des électrodes (20, 21) portées par chaque face du disque.

9. Capteur selon la revendication 4, caractérisé en ce que les collecteurs de courant (16, 17) sont constitués par des bandes obtenues par déposition en couche mince ou épaisse d'un matériau conducteur ou par laminage d'un film isolant revêtu d'un matériau conducteur, ledit matériau conducteur étant choisi parmi l'or, l'argent, le platine, le nickel, le palladium, le titane ou le carbone.

10. Capteur selon la revendication 4, caractérisé en ce que le matériau isolant rigide constituant le disque est une résine vinylique, une céramique, le mica, un polyé tylène téréphtalate ou le verre.

11. Capteur selon la revendication 4, caractérisé en ce que le dispositif d'entraînement (6) est constitué par un trou traversant polygonal central, par une couronne dentée, pour un entraînement par engrenage ou par friction, située sur une de ses faces, ou par une dentelure du borde du disque.

12. Capteur selon la revendication 1, caractérisé en ce que le disque comporte en outre des moyens de positionnement des zones de mesure (34) constituées par des encoches (11) régulièrement espacées dans l'épaisseur du pourtour du disque.

13. Capteur selon la revendication 1, caractérisé en ce que le disque comporte en outre sur une de ses faces une portion de nervure radiale (8) destinée à venir coopérer avec un butée située dans l'appareil de mesure empêchant le disque d'effectuer une rotation supérieure à 360°.

14. Capteur selon la revendication , caractérisé en ce que le disque comporte en outre un cliquet anti-retour (9).

15. Capteur selon la revendication 9, caractérisé en ce qu'un collecteur de courant (16) est en platine et constitue une électrode de travail (21) et que l'autre collecteur de courant (17) est en argent/chlorure d'argent et constitue une électrode de référence (20).

16. Capteur selon la revendication 15, caractérisé en ce que l'électrode de travail (21) est en outre revêtue d'un réactif (26) comportant au moins une enzyme spécifique du composant présent dans la solution.

17. Capteur selon la revendication 16, caractérisé en ce que le réactif (26) comporte en outre un médiateur.

18. Capteur selon la revendication 1, caractérisé en ce que le disque a une épaisseur comprise entre 0,5 et 6 mm, un diamètre compris entre 5 et 15 cm, de préférence entre 6 et 12 cm, et qu'il comporte entre 6 et 24 zones actives (34), de préférence entre 8 et 12 zones actives.

19. Capteur selon la revendication 1, caractérisé en ce qu'il comporte en outre un boîtier (40), pourvu d'ouvertures donnant accès à une zone de mesure (34) et à sa zone d'isolement associée (7), aux zones de contact (23,24) et aux moyens d'entraînement (6), ledit boîtier étant destiné à protèger et à positionner ledit capteur dans un appareil de mesure.
